(19) Europäisches Patentamt
European Patent Office
Office européen des brevets

(11) Veröffentlichungsnummer: **0 201 828**
A2

(12) **EUROPÄISCHE PATENTANMELDUNG**

(21) Anmeldenummer: **86106112.5**

(22) Anmeldetag: **05.05.86**

(51) Int. Cl.⁴: **A 61 L 15/03**
A 61 K 47/00, A 61 K 9/22

(30) Priorität: **11.05.85 DE 3517080**

(43) Veröffentlichungstag der Anmeldung:
**20.11.86 Patentblatt 86/47**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **von Bittera, Miklos**
**Max-Scheler-Strasse 7**
**D-5090 Leverkusen 3(DE)**

(72) Erfinder: **Meyer, Rolf-Volker, Dr.**
**Buchheimer Strasse 23**
**D-4150 Krefeld(DE)**

(72) Erfinder: **Beermann, Dieter, Dr.**
**Cordulastrasse 18**
**D-5600 Wuppertal 1(DE)**

(54) **Komponente für therapeutische Wirkstoffabgabesysteme.**

(57) In therapeutischen Wirkstoffabgabesystemen auf Basis von Polymeren mit Kautschukeigenschaften wird als Komponente ein Poly-N-vinyllactam und/oder ein Copolymer eines N-Vinyllactams eingesetzt.

EP 0 201 828 A2

0201828

BAYER AKTIENGESELLSCHAFT 5090 Leverkusen, Bayerwerk
Konzernverwaltung RP
Patentabteilung Mn/by-c

## Komponente für therapeutische Wirkstoffabgabesysteme

Die Erfindung betrifft Poly-N-vinyllactame oder Copolymere der N-Vinyllactame als Komponenten für therapeutische Wirkstoffabgabesysteme auf Basisvon Polymeren mit Kautschukeigenschaften, insbesondere für medizinische Pflaster.

Die erfindungsgemäßen Wirkstoffabgabesysteme bestehen im allgemeinen aus einer undurchlässigen Deckschicht, einer polymeren Wirkstoffreservoirschicht und einer im wesentlichen undurchlässigen, abziehbaren Schutzschicht.

Bekannte medizinische Pflaster enthalten in der Reservoirschicht Wirkstoffe, die im allgemeinen unter Lager- und Anwendungsbedingungen flüssig sind. Feste Wirkstoffe müssen für die Anwendung in nichtkristalliner Form in der Wirkstoffreservoirschicht vorliegen. Sie kristallisieren jedoch nach relativ kurzer Zeit aus und werden nur in geringem Maß oder ungleichmäßig von der Haut resorbiert.

Es wurde eine Komponente für therapeutische Wirkstoffabgabesysteme auf Basis von Polymeren mit Kautschukeigenschaften gefunden, die Polymere von Vinyllactamen und/oder Copolymere der N-Vinyllactame enthält.

Die erfindungsgemäße Komponente kann einen oder mehrere der Poly-N-vinyllactame und/oder der Copolymeren der N-Vinyllactame enthalten.

Überraschenderweise bewirkt die erfindungsgemäße Komponente eine hohe Lagerstabilität von therapeutischen Wirkstoffabgabesystemen mit festen Wirkstoffen. Die Wirkstoffe können in hohen Dosen eingesetzt werden. Sie werden langsam und gleichmäßig an die Haut abgegeben. Eine Kristallisation der Wirkstoffe tritt nicht oder sehr verzögert ein. Mit Hilfe der erfindungsgemäßen Komponenten ist es möglich, auch feste Wirkstoffe in befriedigender Weise therapeutischen Wirkstoffabgabesystemen auf Basis von Polymeren mit Kautschukeigenschaften zugänglich zu machen.

Überraschenderweise wurde gefunden, daß die erfindungsgemäßen Komponenten in therapeutischen Wirkstoffabgabesystemen auf Basis von Polymeren mit Kautschukeigenschaften, die Kristallisationsneigung kristalliner Wirkstoffe verhindern oder zumindest stark verzögern.

Erfindungsgemäß werden Polymere der N-Vinyllactame der Formel

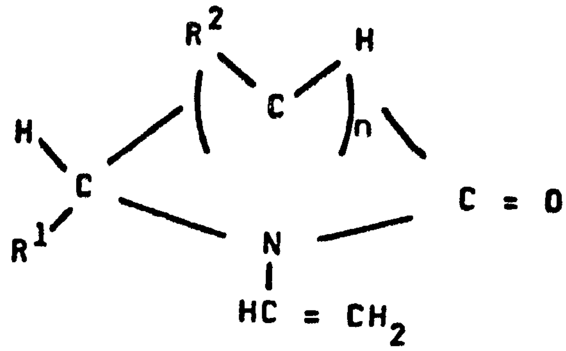

(I)

in der

$R^1$ und $R^2$ gleich oder verschieden sind und Wasserstoff oder Niederalkyl bedeuten und

n einen Wert von 2 bis 10 bedeutet,

bevorzugt.

Insbesondere bevorzugt werden polymere N-Vinyllactame nach Formel (I)

bei denen

$R^1$ und $R^2$ gleich oder verschieden sind und Wasserstoff, Methyl oder Ethyl bedeuten und

n für eine der Zahlen 2, 3, 4 oder 10 steht.

Im einzelnen seien die folgenden monomeren, zur Herstellung der erfindungsgemäß einzusetzenden (Co)polymeren geeigneten N-Vinyllactame genannt: N-Vinylpyrrolidon, N-Vinylpiperidon, N-Vinylcaprolactam, N-Vinyl-3-methylpyrrolidon, N-Vinyl-3-methylpiperidon, N-Vinyl-3-methylcaprolactam, N-Vinyl-4-methylpyrrolidon, N-Vinyl-4-methylpiperidon, N-Vinyl-4-methylcaprolactam, N-Vinyl-5-methylpyrrolidon, N-Vinyl-4,5-dimethylpyrrolidon,

N-Vinyl-3-ethylpyrrolidon, N-Vinyl-5,5-dimethylpyrrolidon, N-Vinyl-3,3,5-trimethylpyrrolidon, N-Vinyl-5-methyl-5-ethylpyrrolidon, N-Vinyl-3,4,5-trimethyl-3-ethylpyrrolidon, N-Vinyl-6-methyl-2-piperidon, N-Vinyl-6-ethyl-2-piperidon, N-Vinyl-3,5-dimethyl-2-piperidon, N-Vinyl-4,4-dimethyl-2-piperidon, N-Vinyl-3,5-dimethyl-2-piperidon, N-Vinyl-4,4-dimethyl-2-piperidon, N-Vinyl-7-methyl-caprolactam, N-Vinyl-7-ethyl-caprolactam, N-Vinyl-3,5-dimethyl-caprolactam, N-Vinyl-4,6-dimethyl-caprolactam und N-Vinyl-3,5,7-Trimethyl-caprolactam.

Als Comonomere zur Herstellung von Copolymeren der N-Vinyllactame seien Acrylverbindungen der Formel

$$CH_2 = C \begin{cases} R^3 \\ X \end{cases} \qquad \text{(II)},$$

in der

$R^3$ Wasserstoff oder Niederalkyl bedeutet und

X Aryl, Halogen oder eine der Gruppen $-O-R^4$, $-O-\overset{O}{\overset{\|}{C}}-R^5$ oder $-\overset{O}{\overset{\|}{C}}-OR^6$ bedeutet,

in denen

$R^4$ für Niederalkyl steht,

$R^5$ für Wasserstoff oder Niederalkyl steht und

$R^6$ für Wasserstoff oder einen Alkylrest mit 1 bis 18 Kohlenstoffatomen steht, genannt.

Die erfindungsgemäßen Komponenten sind Copolymere von N-Vinyllactamen mit 0 - 30 Gew.-%, bevorzugt 0 - 10 % Gew.-%, mit mindestens einer der Acrylverbindungen.

Halogen bedeutet erfindungsgemäß Fluor, Chlor, Brom und Iod, bevorzugt Fluor und Chlor.

Ein Alkylrest bedeutet erfindungsgemäß einen geradkettigen oder verzweigten Kohlenwasserstoffrest mit 1 bis 18, bevorzugt 1 bis 12,Kohlenstoffatomen. Insbesondere bevorzugt ist Niederalkyl mit 1 bis etwa 6, Kohlenstoffatomen. Beispielsweise seien die folgenden Alkylreste genannt: Methyl, Ethyl, Propyl, Isopropyl, Butyl, Isobutyl, Pentyl, Isopentyl, Hexyl, Isohexyl und Stearyl.

Als Beispiele für die Comonomeren seien genannt: (Meth)Acrylamid, (Meth)Acrylester mit 1 bis 18 Kohlenstoffatomen in der Alkoholkomponente, wie Methyl(meth)acrylat, Ethyl(meth)- acrylat, n-Butyl(meth)acrylat, N-Hexyl(meth)acrylat, 2-Ethylhexyl-(meth)-acrylat, Lauryl(meth)acrylat, Stearyl(meth)acrylat, Vinylester wie z.B. Vinylacetat, Vinylpropionat, Vinylbutyrat, Vinylether wie z.B. Methylvinylether, Ethylvinylether, n-Butyl-vinylether und Vinylchlorid, Styrol und α-Methylstyrol.

Vorzugsweise werden polymere N-Vinylpyrrolidone und N-Vinylcaprolactame eingesetzt, wobei als Comonomere vorzugsweise Vinylacetat und/oder Alkyl(meth)acrylate, bevorzugt Ethylhexylacrylat und/oder Laurylmethacrylat und/oder Stearylacrylat, mit einpolymerisiert sein können.

Besonders bevorzugt werden N-Vinylpyrrolidon-Homopolymere eingesetzt.

Die erfindungsgemäßen Komponenten können Copolymere in einer Menge von 0 bis 30 Gew.-%, bevorzugt 0 bis 10 Gew.-%, neben den Polymeren der N-Vinyllactame enthalten.

Die Polymeren von N-Vinyllactamen oder Copolymeren von N-Vinyllactamen haben im allgemeinen ein mittleres Molekulargewicht von etwa 10 000 bis 750 000, vorzugsweise von 20 000 bis 250 000.

Die Herstellung der genannten Polymere und die Bestimmung der Molekulargewichte kann nach bekannten Methoden erfolgen (Monographien in Chemie Ingenieur-Technik 66, (1954), DE-AS 12 68 391, US 44 82 534).

Die Erfindung betrifft auch ein therapeutisches Wirkstoffabgabesystem umfassend eine Deckschicht, die im wesentlichen für den Wirkstoff undurchlässig ist, eine Wirkstoffreservoirschicht und eine abziehbare Schutzschicht, die im wesentlichen für den Wirkstoff undurchlässig ist, das dadurch gekennzeichnet ist, daß die Wirkstoffreservoirschicht bis zu 30 Gew.-% Wirkstoff in eines Systems von Polymeren mit Kautschukeigenschaften, eine erfindungsgemäße Komponente aus Polymeren von

N-Vinyllactamen und/oder von Copolymeren von N-Vinyllactamen und an sich bekannte Hilfsstoffe enthält.

Die polymere Basis in der Wirkstoffreservoirschicht sind im allgemeinen Polymere mit Kautschukeigenschaften. Erfindungsgemäße Polymere mit Kautschukeigenschaften können beispielsweise Polyisobutylene und/oder Copolymerisate des Isobutylens, Dienkautschuke mit mindestens 30 % Cis-1,4-Verknüpfung und Mooney-Viskositäten von 20 bis 100 (ML 1+4 bei 100°C) sowie überwiegend amorphe (Co)- Polymere mit mindestens einem α-Olefin oder einem Cycloolefin, vorzugsweise von zwei verschiedenen $C_2$ bis $C_{18}$- α-Olefinen, die auch mit einem weiteren Diolefin copolymerisiert sein können und Glastemperaturen von $< 20°C$ haben, sein. Die Polymeren können selbstverständlich jeweils für sich oder auch in Abmischungen mehrerer Polymerer miteinander eingesetzt werden.

Als erfindungsgemäße Polyisobutylene werden Polyisobutylene verstanden, die herstellungsbedingt eine Molmassen-Verteilung $M_w/M_n$ von 1,5 bis 3,5, vorzugsweise 2,0 bis 3,0, und ein Viskositätsmittel der Molmasse von 30 000 bis zu 4 000 000 g/Mol aufweisen. Vorzugsweise beträgt das Viskositätsmittel der erfindungsgemäß einzusetzenden Polyisobutylene 50 000 bis 1 000 000 g/Mol, besonders bevorzugt 80 000 bis 500 000 g/Mol. Die Viskositätsmittel lassen sich in an sich bekannter Weise bestimmen (Polymer-Handbook, J. Brandrup und F.H. Immergut Wiley u. Sons, New York, 1975, Kap. IV, Seite 35).

Die genannten Polyisobutylene sind an sich bekannt und können beispielsweise gemäß US 22 03 873 oder gemäß DR-P 704 038 mit Hilfe von sauren Katalysatoren hergestellt werden.

Erfindungsgemäße Copolymerisate des Isobutylens sind beispielsweise Copolymerisate des Isobutylens mit 0,5 bis 5 Mol-% konjugierten Diolefinen, vorzugsweise solche mit 4 bis 6 Kohlenstoffatomen, wie z.B. Butadien-1,3, Piperylen, 2,3-Dimethylbutadien, besonders bevorzugt mit Isopren, deren Molmassen von 30 000 bis 200 000 g/Mol betragen können.

Die Isobutylencopolymere sind an sich bekannt (Houben-Weyl, Methoden der organischen Chemie, Band 14/1, S. 629 (1961); H. Güterbock "Isobutylen und Isobutylen-Mischpolymerisate", Springer Verlag, Berlin, Göttingen, Heidelberg 1959, S. 108 ff.).

Ganz besonders bevorzugt werden Polyisobutylen-Homopolymerisate mit einem Viskositätsmittel von 80 000 bis 500 000.

Als erfindungsgemäße Dienkautschuke seien beispielsweise Produkte auf Basis von 1,3-Dienen wie Butadien, Isopren, Piperylen, 2,3-Methylbutadien, vorzugsweise von Butadien, genannt. Die Dienkautschuke sind an sich bekannt; ihre Herstellung kann durch Wahl des Metallkatalysators variiert werden (Ullmanns Encyclopädie der technischen Chemie, 4. Auflage, Band 13, Seite 602 bis 611, Verlag Chemie, Weinheim/New York (1977)).

Bevorzugt werden Dienkautschuke mit über 80 % cis-1,4-Verknüpfung eingesetzt. Auch Naturkautschuk ist im Rahmen der genannten Kennzahlen geeignet.

Zur Kombination mit den Dienkautschuken geeignete Vinylaromaten sind z.B. Styrol, α-Methylstyrol, Vinyltoluole, p-Ethylstyrol, Dimethyl-styrole, 4-Vinyldiphenyl, vorzugsweise Styrol. Auch die mit Vinylaromaten modifizierten Dienkautschuke sind an sich bekannte Polymere ("Styrol-Butadien-Kautschuk") und können nach bekannten Verfahren hergestellt werden. Nach den bekannten Verfahren ist es möglich, den vinylaromatischen Anteil im Polymeren nicht nur statistisch sondern auch ganz oder teilweise als Blockstruktur in den Dienkautschuk einzubauen.

Die erfindungsgemäßen Dienkautschuke haben im allgemeinen eine mittlere Molmasse von 20 000 bis 2 000 000, vorzugsweise von 25 000 bis 500 000, g/Mol.

Beispiele für die überwiegend amorphen (Co)-Polymeren sind amorphes Polypropylen (überwiegend ataktisch), amorphes Polybuten-1 (überwiegend ataktisch), Polyoctenamere und Ethylen-Propylen-Copolymere, die die minderanteilige Komponente entweder statistisch oder in Blöcken, vorzugsweise in Blöcken eingebaut enthalten. Solche überwiegend amorphen (Co)-Polymeren werden z.B. in Angew. Chem. 73, 186 (1961) beschrieben.

Weitere Polymere mit Kautschukeigenschaften sind beispielsweise Copolymerisate des Ethylens und/oder Propylens mit weiteren $C_4$ bis $C_{18}$-α-Olefinen, vorzugsweise des Ethylens mit $C_4$ bis $C_{12}$-α-Olefinen.

Beispiele für mit Dienen copolymerisierte α-Olefin-Copolymere sind als EPDM-Kautschuke bekannte Produkte, die vorzugsweise aus 20 bis 90 Gew.-Teilen Ethylen, 10 bis 80 Gew.-Teilen Propylen und 2 bis 15 Gew.-Teilen, bevorzugt 4 bis 10 Gew.-Teilen eines nicht konjugierten Diens bestehen. Als Dienkomponente in den EPDM-Kautschuken sind aus der Vielzahl möglicher Diene Dicyclopentadien, Ethylidennorbornen und Hexadien-1,4 besonders bevorzugt.

Besonders bevorzugte erfindungsgemäß zu verwendende EPDM-Polymere sind solche mit Molekulargewichten von 20 000 g/Mol bis $1 \times 10^6$ g/Mol, vorzugsweise von 25 000 bis 500 000 g/Mol.

Die erfindungsgemäß zu verwendenden Polymeren mit Kautschukeigenschaften können für sich allein oder in Mischungen mit mehreren der beschriebenen Polymeren eingesetzt werden.

Ganz besonders bevorzugte Polymere mit Kautschukeigenschaften sind Polybutadien-Kautschuke mit Mooney-Viskesitäten von 30 bis 60 (ML 1+4 bei 100°C). Diese können gegebenenfalls mit 30 bis 70 Gew.-% Polyisobutylen, das eine Molmassenverteilung $M_w/M_n$ von 2,0 bis 3,0 aufweist, abgemischt werden.

In die Wirkstoffreservoirschicht können flüssige und feste, bevorzugt feste, Wirkstoffe aufgenommen werden.

Die Wirkstoffreservoirschicht der erfindungsgemäßen therapeutischen Wirkstoffabgabesysteme kann bis zu 30, bevorzugt 1 bis 20 Gew.-%, Wirkstoff enthalten, bezogen auf die Gesamtmasse.

Als Wirkstoffe seien beispielsweise Antiphlogistika der Formel

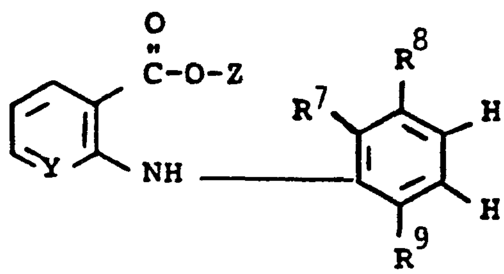

(III),

in der

$R^7$, $R^8$ und $R^9$ gleich oder verschieden sind und Wasserstoff, Chlor, $C_1$ bis $C_4$-Alkyl oder Trifluormethyl bedeuten,

Y Stickstoff oder eine CH-Gruppe bedeutet und

Z Wasserstoff oder gegebenenfalls substituiertes $C_1$ bis $C_4$-Alkyl bedeutet,

genannt.

Bevorzugt sind Antiphlogistika der Formel (III), in welcher

$R^7$, $R^8$ und $R^9$ gleich oder verschieden sind und Wasserstoff, Methyl, Trifluormethyl oder Chlor bedeuten,

Y für eine CH-Gruppe steht und

Z Wasserstoff oder Hydroxy-alkoxy-alkyl mit 1 bis 6 Kohlenstoffatomen bedeutet.

Besonders bevorzugt sind die folgenden Antiphlogistika:

N-(α,α,α-Trifluor-m-tolyl)-anthranilsäure = Flufenaminsäure

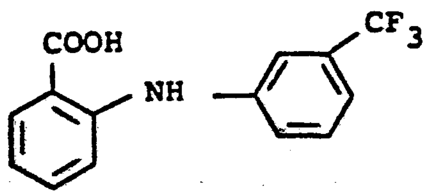

N-(2,3-Xylyl)-anthranilsäure

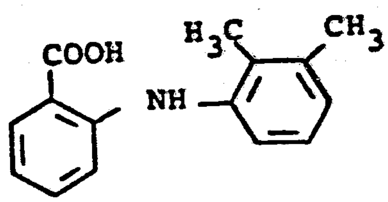

2-(2,6-Xylidino)-nicotinsäure

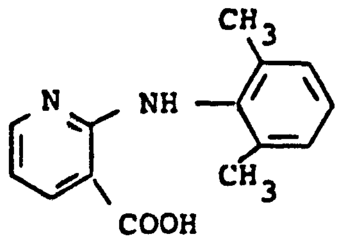

Es seien außerdem Antiphlogistika der Formel

$$Ar-(\overset{\displaystyle O}{\overset{\|}{-C-}})_o-(\overset{\displaystyle R^{10}}{\overset{|}{CH}})_p-(CH_2)_q-COOH \qquad (IV),$$

in der

$R^{10}$ Wasserstoff, gegebenenfalls substituiertes Alkyl,

Ar Aryl, Heteroaryl, substituiertes Aryl, substituiertes Heteroaryl und

o und p 0, 1 oder 2 bedeuten und

q 0 oder 1 bedeutet,

genannt.

Im Rahmen der Formeln (III) und (IV) kann Alkyl einen geradkettigen oder verzweigten Alkylrest mit 1 bis 4 Kohlenstoffatomen, beispielsweise Methyl, Ethyl, Propyl, Isopropyl, Butyl und Isobutyl, bedeuten, der gegebenenfalls durch Alkoxyalkyl oder Trihalogenalkyl substituiert sein kann.

Aryl bzw. Heteroaryl kann beispielsweise Phenyl, Naphthyl, Thiophenyl, Pyrollyl, Indenyl, Indolyl, Benzthiazinyl oder Phenothiazinyl bedeuten.

Substituenten für Aryl bzw. Heteroaryl können Alkyl, bevorzugt geradkettiges oder verzweigtes Alkyl mit 1 bis 6 Kohlenstoffatomen, Alkoxy ($C_1$-$C_6$), Oxalkyl ($C_1$ bis $C_6$), Acyl ($C_1$ bis $C_6$), Hydroxyl, Acetoxy ($C_1$ bis $C_6$), Benzoyl, durch Niederalkyl-substituiertes Benzoyl, Phenyl, durch Niederalkyl-substituiertes Phenyl, Phenoxy, Halogen, Phenyl-alkenyl ($C_1$ bis etwa $C_6$) oder Phenyl-alkyl ($C_1$ bis etwa $C_6$) sein.

Im Rahmen der Formel (IV) seien die folgenden Antiphlogistika genannt:

2-Hydroxybenzoesäure

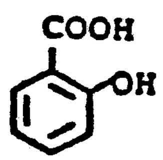

2-Acetoxybenzoesäure

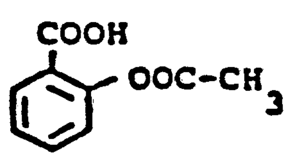

0201828

2',4'-Difluor-4-hydroxy-3-biphenylcarbonsäure

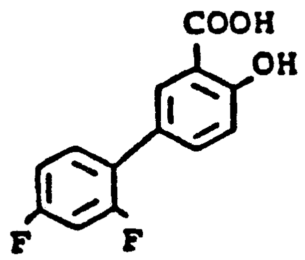

4-Allyloxy-3-chlorphenylessigsäure = Alclofenac

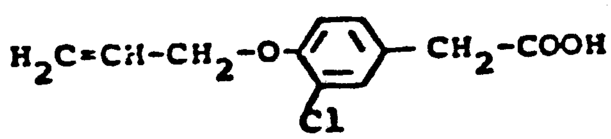

2-[(2,6-Dichlorphenyl)-amino]-phenylessigsäure

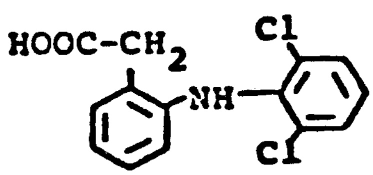

10-Methyl-phenothiazin-2-yl-essigsäure = Metiazinsäure

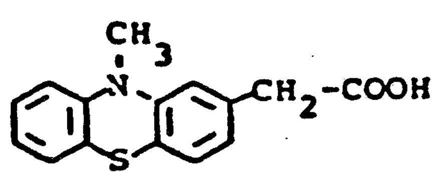

2-(5-Benzoyl-2-thienyl)-propionsäure

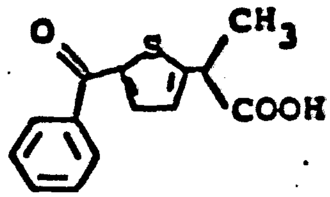

1-(p-Chlorbenzoyl)-5-methoxy-2-methylindol-3-essigsäure = Indometacin

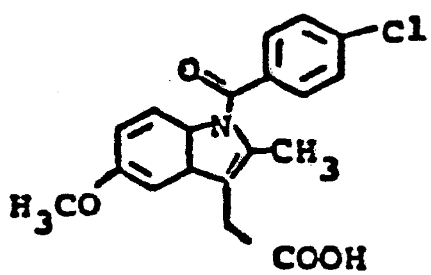

1-(p-Chlorbenzoyl)-5-methoxy-2-methylindol-3-acetoxy-essigsäure = Acemetacin

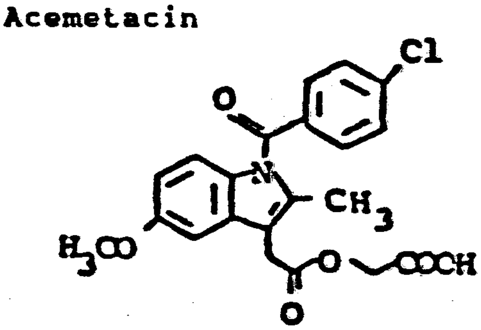

(Z)-5-Fluor-2-methyl-1-([(4-methylsulfinyl)phenyl]-methylen)-1H-inden-3-essigsäure

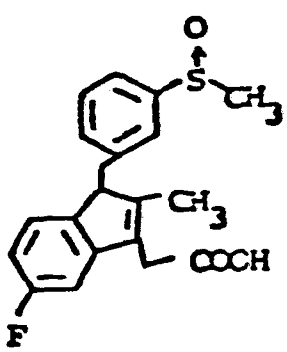

4-Butyl-1,2-diphenyl-3,5-pyrazolidin-dion = Phenylbutazon

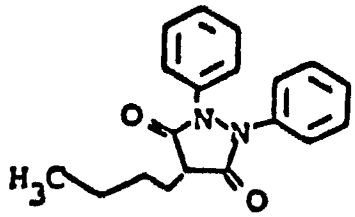

D-2-(6-Methoxy-2-naphthyl)-propionsäure = Naproxen

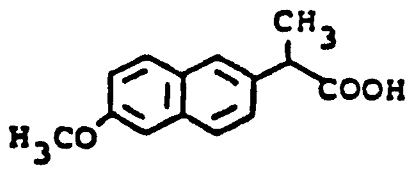

2-(p-Isobutylphenyl)-propionsäure

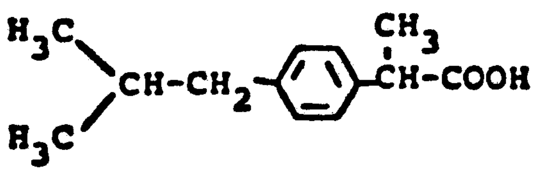

2-(3-Phenoxyphenyl)-propionsäure

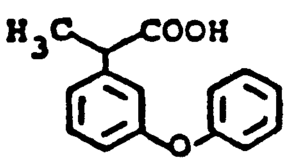

2-(m-Benzoylphenyl)-propionsäure = Ketoprofen

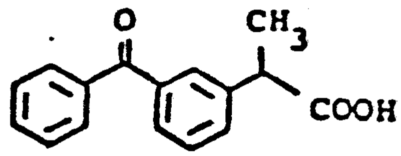

2-[4-(1-Oxo-2-isoindolinyl)-phenyl]-propionsäure = Indoprofen

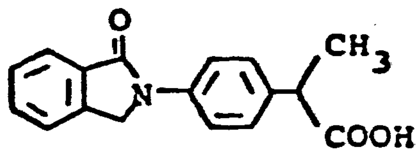

2-(2-Fluorbiphenyl-4-yl)-propionsäure

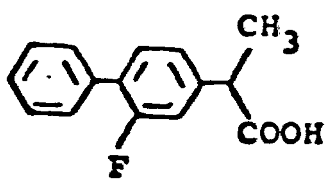

3-(4-Biphenylcarbonyl)-propionsäure

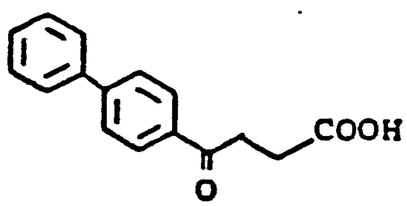

1-Methyl-5-(p-toluoyl)-pyrrol-2-yl-essigsäure

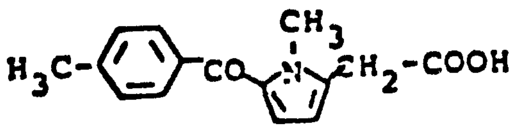

4-(3-Methyl-but-2-enyl)-1,2-diphenyl-pyrazolidin-3,5-dion = Feprazon

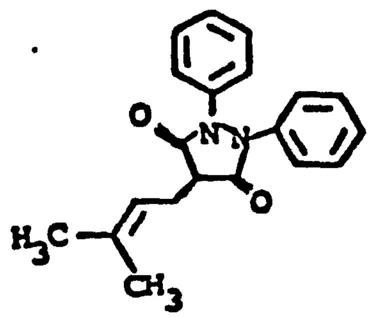

2-(4-Chlorphenyl)-2-methyl-5-benzoxazol-essigsäure = Benoxaprofen

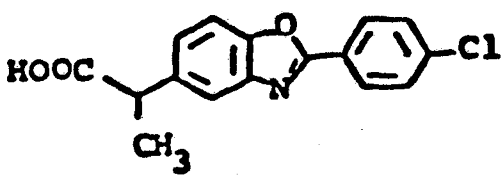

4-Hydroxy-2-methyl-N-2-thiazolyl-2H-1,2-benzothiazin-3-carboxamid-1,1-dioxid

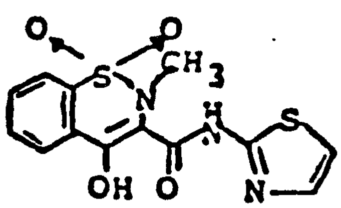

4-Hydroxy-2-methyl-N-2-pyridinyl-2H-1,2-benzothiazin-3-carboxamid-1,1-dioxid

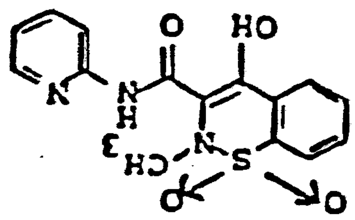

Besonders bevorzugte Antiphlogistika sind Ketoprofen und Acemetacin.

Die Wirkstoffe konnten sowohl einzeln als auch im Gemisch in die therapeutischen Wirkstoffabgabesysteme eingearbeitet werden.

Den Wirkstoffen können zusätzlich noch weitere Wirksubstanzen zugesetzt werden. Es ist auch möglich, kühlende oder duftabgebende Substanzen beizumischen.

Beispielsweise seien Methylsalicylat, Glykolsalicylat, Salicylsäure, Menthol, Pfefferminzöl, Kampfer, Thymol, Acrinol, Scopoliaextrakt, Chlorpeniraminmaleat, Benzylnicotinat, Capsicumextrakt, Nonylvanillylamid und Capsaicin genannt.

Erforderlichenfalls können die erfindungsgemäßen therapeutischen Wirkstoffabgabesysteme Additive und Füllstoffe, z.B. Alterungsschutzmittel, Antioxydantien und Verstärkungsfüllstoffe enthalten, soweit die nichtkristalline Struktur des Systems nicht zerstört wird.

Als weitere Hilfsstoffe für die Wirkstoffreservoirschicht seien Schleppmittel wie Öle, Fettsäureester, Triglyceride, Alkohole und/oder Fettsäuren genannt.

Unter Ölen im Sinne der vorliegenden Erfindung werden hochsiedende, aliphatische, araliphatische und/oder aromatische Kohlenwasserstoffe verstanden, vorzugsweise Paraffinöl, Purcellinöl, Perhydrosqualen und Lösungen von mikrokristallinen Wachsen in den Ölen, Mineralöle, bevorzugt Öle, deren Siedebereich zwischen 150° C und 400° C liegt; ferner ungesättigte Kohlenwasserstoffe mit mindestens 16 Kohlenstoffatomen wie z.B. Oligomere von Monoolefinen wie Tetraisobutylen, Pentaisobutylen, Hexaisobutylen oder auch flüssige Polymerisate aus Dien-(Monoen)-(Co)-Polymerisaten. Beispiele für flüssige Polymerisate aus konjugierten Dienen sind solche aus Butadien, Isopren, 1,3-Pentadien, 2,3-Dimethylbutadien, Copolymerisate verschiedener Diene sowie auch flüssige Copolymerisate aus einem konjugierten Diolefin und geringen Mengen von Monoolefin wie z.B. Buten-1, Isobuten, Hexen-1, Octen-1, Styrol mit einem Molekulargewicht

von 400 bis 6000, vorzugsweise 800 bis 3000, Iodzahlen von 200 bis 500 und Viskositäten von 100 bis 10 000 cP bei 50°C.

Besonders bevorzugt sind flüssige Polybutadien-Polymerisate, die zumindestens 90 Gew.-% 1,4-verknüpft sind, deren Anteil an cis-Doppelbindung mehr als 60 % beträgt und deren Molmassen 1000 bis 4000 betragen.

Unter Ölen werden auch Silikonöle verschiedener Viskosität, vorzugsweise mit mittleren Molekulargewichten von 312 bis 15 000, besonders bevorzugt Polydimethylsiloxane, verstanden.

Unter Fettsäureestern werden solche verstanden, die mindestens 12 Kohlenstoffatome, vorzugsweise 15 bis 46 Kohlenstoffatome, besonders bevorzugt 16 bis 36 Kohlenstoffatome, enthalten. Insbesondere werden darunter verstanden: Ethylstearat, Laurinsäurehexylester, Dipropylen-glykolpelargonat, Palmitinsäurecetylester, Isopropylmyristat, Isopropylpalmitat, Caprylsäureester und Caprinsäureester von gesättigten Fettalkoholen der Kettenlänge $C_{12}$ bis $C_{18}$, Isopropylstearat, Ölsäureoleylester, Ölsäuredodecylester, Ethyloleat und künstliches Entenbürzeldrüsenfett.

Unter Triglyceriden werden reine oder gemischte Ester des Glycerins mit Fettsäuren der Kettenlänge $C_8$ bis $C_{18}$ verstanden, vorzugsweise Capryl- und/oder Caprinsäuretriglyceride.

Unter Fettsäuren werden gesättigte oder ungesättigte Fettsäuren, vorzugsweise solche mit 12 bis 24 Kohlenstoffatomen, einzeln oder im Gemisch miteinander, besonders bevorzugt Ölsäure, verstanden. Unter Ölen im Sinne der Erfindung werden ferner verstanden: Süßmandelöl, Avocadoöl, Sesamöl, Ricinusöl, Olivenöl, Traubenkernöl, Nelkenöl, Erdnußöl, Maisöl, Haselnußöl, Jojobaöl, Carthamaöl und Weizenkeimöl, jeweils einzeln oder im Gemisch.

Weitere Hilfsstoffe für die Wirkstoffreservoirschicht sind klebrig-machende Stoffe wie beispielsweise Kolophonium, dehydriertes Kolophonium, Glycerinester von dehydriertem Kolophonium, Glycerinester von Kolophoniumgummi, hydriertes Collophonium, Glycerinester von hydriertem Kolophonium, Pentaerythritester von hydriertem Kolophonium, Methylester von hydriertem Kolophonium, polymerisiertes Kolophonium, Glycerinester von polymerisiertem Kolophonium, Terpenharze, Cumaron/Inden-harze, hydrierte Petroleumharze, mit Maleinsäureanhydrid modifiziertes Kolophonium und Kolophoniumderivate, $C_5$-Petroleumharze und Halbester von Styrol/Maleinsäure-Co-Polymeren einzeln oder im Gemisch miteinander.

Besonders bevorzugte klebrig-machende Stoffe sind Polyterpenharze aus α- bzw. β-Pinen oder modifizierte Glycerinester des Kolophoniums. Diese Harze können je nach den erforderlichen Eigenschaften hinsichtlich der Klebrigkeit und der Haftfestigkeit auf dem Teil, an dem das resultierende therapeutische Wirkstoffabgabesystem angebracht werden soll, entweder allein oder in Kombination miteinander verwendet werden.

Weitere Bestandteile der therapeutischen Wirkstoffabgabesysteme sind die Deckschicht und die im wesentlichen undurchlässige, abziehbare Schutzschicht. Beide Komponenten sind an sich bekannt.

Die Deckschicht kann vorzugsweise aus einem längs-querelastischen, mit einem Polymer imprägnierten oder beschichteten textilen Flächengebilde, insbesondere Gewirk oder Gestrick, bestehen.

Die im wesentlichen undurchlässige, abziehbare Schutzschicht kann aus okklusiven, flexiblen oder nichtflexiblen Materialien bestehen, wie Polyethylen, Polypropylen, Polyethylenterephthalat, Nylon. Als Abziehfolie können auch Metallfolien, wie Aluminiumfolie, allein oder mit Polymeren laminiert, angewandt werden. Auch mehrschichtige Folien, wie Laminate aus Polyethylen mit Polyester-PE-terephthalat und mit Aluminium bedampft, können eingesetzt werden. Andere abziehbare Folien sind z.B. mit Silikon behandelte Polyester, Polyethylenterephthalat mit endständigen Silikongruppen, behandeltes Papier, mit Silikon behandeltes Papier oder mit Polyethylen beschichtetes Papier.

Therapeutische Wirkstoffabgabesysteme sind beispielsweise medizinische Pflaster.

Die erfindungsgemäßen therapeutischen Wirkstoffabgabesysteme enthalten in der Wirkstoffreservoirschicht 0,5 bis 10 Gew.-%, bevorzugt 1,0 bis 6 Gew.-% der erfindungsgemäßen Komponente aus einem Poly-N-vinyllactam

und/oder einem Copolymeren der N-Vinyllactame.

Die erfindungsgemäßen therapeutischen Wirkstoffabgabesysteme können hergestellt werden, indem man die Lösung aus einer polymeren Basis, der erfindungsgemäßen Komponente und an sich bekannten Hilfsstoffen und die Lösung eines Wirkstoffs mischt, wobei der Anteil des Wirkstoffs bis zu 30 Gew.-% bezogen auf die polymere Basis beträgt, das Gemisch gleichmäßig auf eine undurchlässige Deckschicht aufträgt und zu einem Film auszieht, die beschichtete Deckschicht trocknet und gegebenenfalls die getrocknete Schicht auf der beschichteten Seite mit einer für den Wirkstoff im wesentlichen undurchlässigen abziehbaren Schutzschicht versieht.

Vorteilhafterweise lassen sich die erfindungsgemäßen Wirkstoffabgabesysteme lange ohne Wirkungsverlust lagern. Die Wirkstoffabgabesysteme geben die Wirkstoffe langsam und gleichmäßig an die Haut ab. Es ist möglich die Wirkstoffe in hohen Dosen einzusetzen.

Die erfindungsgemäßen Wirkstoffabgabesysteme eignen sich besonders für eine transdermale Applikation der Wirkstoffe.

Beispiel 1

Eine 12,5 %ige Polyisobutylenlösung (M.G. Viskositätsmittel 1.270.000) in Benzin, wird auf silikonisiertes Papier aufgetragen, ein Gewirk bestehend aus Polyamid-Polyurethanfasern aufkaschiert. Dann wird im Trockenkanal stufenweise bei 70/90/110° C getrocknet (Polymer-Sperrschicht, 100-150 g/m$^3$ I).

Auf silikonisiertem Papier wird eine in Benzin/Aceton gelöste Mischung bestehend aus

| | |
|---|---|
| 36,000 g | Polyisobutylen M.G. Viskositätsmittel 1.270.000 |
| 36,000 g | Paraffin dünnflüssig, |
| 4,000 g | Isopropylmyristat, |
| 9,000 g | Polyterpenharz aus β-Pinen, |
| 10,000 g | Ketoprofen, |
| 5,000 g | Polyvinylpyrrolidon (mittleres Molgewicht ca. 25 000) |

aufgetragen und im Trockenkanal stufenweise bei 70/90/100° C getrocknet (Wirkstoffabgabesystem, 75-150 g/m$^2$ II).

Nach dem Trocknen wurde das mit Polyisobutylen imprägnierte Gewirk mit Stretchcharakter (I) auf das Wirkstoffabgabesystem (II) zukaschiert.
Freisetzung: 80,5 % in 24 Stunden.

Beispiel 2

Eine 12,5 %ige Polyisobutylenlösung (M.G. Viskositätsmittel 1.270.000) in Benzin, wird auf silikonisiertes Papier aufgetragen, ein Gewirk bestehend aus Polyamid-Polyurethanfasern aufkaschiert und im Trockenkanal stufenweise bei 70/90/110°C getrocknet (Polymer-Sperrschicht, 100-150 g/m³ I).

Auf silikonisiertem Papier wird eine in Benzin/Aceton gelöste Mischung bestehend aus

| | | |
|---|---|---|
| 36,000 | g | Polyisobutylen M.G. Viskositätsmittel 400 000 |
| 36,000 | g | Paraffin dünnflüssig, |
| 9,000 | g | Polyterpenharz aus β-Pinen, |
| 1,000 | g | Isopropylmyristat, |
| 6,000 | g | Laurinsäurehexylester, |
| 10,000 | g | Ketoprofen, |
| 2,00 | g | Polyvinylpyrrolidon (mittleres Molgewicht ca. 25 000) |

aufgetragen und im Trockenkanal stufenweise bei 70/90/100°C getrocknet (Wirkstoffabgabesystem, 75-150 $g/m^2$ II).

Nach dem Trocknen wurde das mit Polyisobutylen imprägnierte Gewirk mit Stretchcharakter (I) auf das Wirkstoffabgabesystem (II) zukaschiert.
Freisetzung: 98 % in 24 Stunden.

Beispiel 3

Auf silikonisiertem Papier wird eine in Benzin/Aceton gelöste Mischung bestehend aus

| | |
|---|---|
| 36,00 g | Polyisobutylen M.G. Viskositätsmittel 1.270.000, |
| 17,000 g | Paraffin dünnflüssig, |
| 10,000 g | Ketoprofen, |
| 9,000 g | Polyterpenharz aus β-Pinen, |
| 1,000 g | Isopropylmyristat, |
| 21,000 g | Laurinsäurehexylester, |
| 1,000 g | Purcellinöl, |
| 5,000 g | Polyvinylcaprolactam (mittleres Molgewicht ca. 22 000) |

aufgetragen und im Trockenkanal stufenweise bei 70/90/100°C getrocknet.

Nach dem Trocknen wurde das Wirkstoffabgabesystem (II) auf mit Polyisobutylen beschichtetes Stretchmaterial (I) kaschiert. (Wie in Beispiel 1 bzw. 2).
Freisetzung: 97,5 % in 24 Stunden.

Beispiel 4

Auf silikonisiertem Papier wird eine in Benzin/Aceton gelöste Mischung bestehend aus

| | |
|---|---|
| 40,000 g | Polyisobutylen M.G. Viskositätsmittel 400 000, |
| 38,000 g | Paraffin dünnflüssig, |
| 9,000 g | Polyterpenharz aus β-Pinen, |
| 1,000 g | Isopropylmyristat, |
| 6,000 g | Laurinsäurehexylester |
| 1,000 g | Polyvinylpyrrolidon (mittleres Molgewicht ca. 25 000), |
| 5,000 g | Ketoprofen |

aufgetragen und im Trockenkanal stufenweise bei 70/90/100° C getrocknet.

Nach dem Trocknen wurde das Wirkstoffabgabesystem (II) auf mit Polyisobutylen beschichtetes Stretchmaterial (I) kaschiert. (Wie in Beispiel 1 und 2). Freisetzung: 98 % in 24 Stunden.

Beispiel 5

Auf silikonisiertem Papier wurde eine Polymerlösung (Benzin/Aceton) bestehend aus

| | |
|---|---|
| 36,000 g | Polyisobutylen M.G. Viskositätsmittel 1.270.000, |
| 35,500 g | Paraffin dünnflüssig, |
| 9,500 g | Polyterpenharz aus α-Pinen, |
| 1,000 g | Isopropylmyristat, |
| 6,000 g | Laurinsäurehexylester, |
| 10,000 g | Acemetacin |
| 2,500 g | Polyvinylpyrrolidon (mittleres Molgewicht ca. 25.000 ) |

aufgetragen und im Trockenkanal stufenweise bei 70/90/110° C getrocknet.

Nach dem Trocknen wurde das Wirkstoffabgabesystem (II) wie in Beispiel 1 und 2 auf beschichtetem Stretchmaterial (I) kaschiert. Freisetzung: 98 % in 24 Stunden des Wirkstoffs.

Wirkstoffabgabesysteme, die kein Polyvinylpyrrolidon als Kristallisationsinhibitor enthalten, setzen ca. 2-10 % Wirkstoff frei.

## Patentansprüche

1. Komponente für therapeutische Wirkstoffabgabesysteme auf Basis von Polymeren mit Kautschukeigenschaften, enthaltend Polymere von N-Vinyllactamen und/oder Copolymere von N-Vinyllactamen.

2. Komponente nach Anspruch 1, enthaltend Polymere von N-Vinyllactamen der Formel

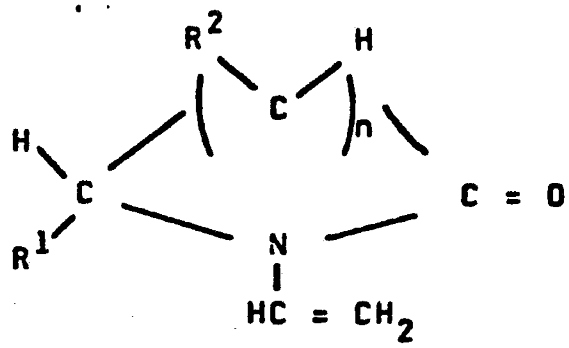

in der

$R^1$, $R^2$ gleich oder verschieden sind und Wasserstoff oder Niederalkyl bedeuten und

n einen Wert von 2 bis 10 bedeutet.

3. Komponente nach Anspruch 1, enthaltend Copolymere aus N-Vinyllactamen der Formel

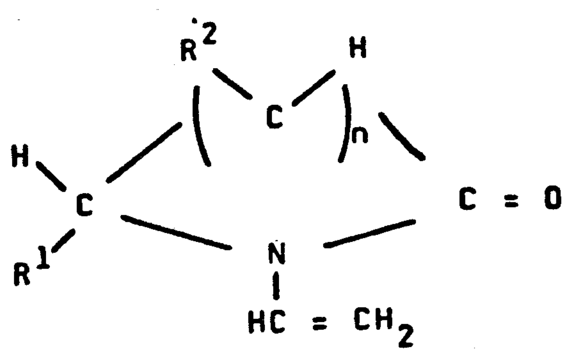

0201828

in der

$R^1$ und $R^2$ gleich oder verschieden sind und Wasserstoff oder Niederalkyl bedeuten und

n einen Wert von 2 bis 10 bedeutet

und mindestens einer Acrylverbindung der Formel

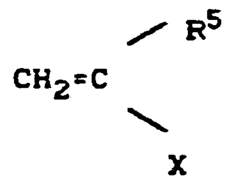

in der

$R^5$ Wasserstoff oder Niederalkyl bedeutet und

X Aryl, Halogen, $-O-R^6$,

$-O-\overset{O}{\overset{\|}{C}}-R^6$ oder $-\overset{O}{\overset{|}{C}}-OR^7$ bedeutet,

in denen

$R^6$ für Wasserstoff oder Niederalkyl steht und

$R^7$ für Wasserstoff oder einen Alkylrest mit 1 bis 18 Kohlenstoffatomen steht.

4. Komponente nach den Ansprüchen 1 bis 3, enthaltend Polymere von N-Vinyllactamen und 0 bis 30 Gew.-% eines Copolymers des N-Vinyllactams mit einer Acrylverbindung.

5. Wirkstoffreservoirschicht für therapeutische Wirkstoffabgabesysteme, enthaltend ein Polymer mit Kautschukeigenschaften, eine Komponente aus Polymeren eines N-Vinyllactams und/oder eines Copolymeren eines N-Vinyllactans, den Wirkstoff und an sich bekannte Hilfsstoffe.

6. Therapeutisches Wirkstoffabgabesystem umfassend eine Deckschicht, die im wesentlichen für den Wirkstoff undurchlässig ist, eine Wirkstoffreservoirschicht und eine abziehbare Schutzschicht, die im wesentlichen für den Wirkstoff undurchlässig ist, dadurch gekennzeichnet, daß die Wirkstoffreservoirschicht neben einer Komponente aus Polymeren eines N-Vinyllactams und/oder eines Copolymeren eines N-Vinyllactams und an sich bekannten Hilfsstoffen bis zu 30 Gew.-% Wirkstoff enthält.

7. Therapeutisches Wirkstoffabgabesystem nach Anspruch 6, dadurch gekennzeichnet, daß es in der Wirkstoffreservoirschicht 0,5 bis 6 Gew.-% einer Komponente aus einem Poly-N-vinyllactam oder einem Copolymeren der N-Vinyllactame enthält.

8. Therapeutisches Wirkstoffabgabesystem, dadurch gekennzeichnet, daß es in der Wirkstoffreservoirschicht als polymere Basis mindestens ein amorphes

Olefinpolymer mit einer Glastemperatur von <20°C enthält.

9. Verfahren zur Herstellung von therapeutischen Wirkstoffabgabesystemen nach den Ansprüchen 6 bis 8, dadurch gekennzeichnet, daß man die Lösung eines Gemischs einer polymeren Basis, einer Komponente aus einem Poly-N-vinyllactam oder einem Copolymeren der N-Vinyllactame und an sich bekannten Hilfsstoffe mit der Lösung eines Wirkstoffs mischt, das Gemisch gleichmäßig auf eine undurchlässige Deckschicht aufträgt und zu einem Film auszieht, die beschichtete Deckschicht trocknet und gegebenenfalls die getrocknete Schicht auf der beschichteten Seite mit einer für die Wirkstoffe im wesentlichen undurchlässigen abziehbaren Schutzschicht versieht, wobei der Anteil des Wirkstoffs bis zu 30 Gew.-%, bezogen auf die Reservoirschicht, beträgt.

10. Verwendung von therapeutischen Wirkstoffabgabesystemen nach den Ansprüchen 6 bis 8 als transdermale Applikationsform.